# EUROPEAN PATENT APPLICATION

(11) **EP 4 389 118 A1**
(43) Date of publication of application: **26.06.2024**
(21) Application number: 22461647.4
(22) Date of filing: 22.12.2022
(51) Int. Cl.: A61K 9/48, A61K 31/00

(54) **DRY POWDER BLEND PHARMACEUTICAL COMPOSITION COMPRISING RAMIPRIL AND INDAPAMIDE**

(71) Applicant: Zaklady Farmaceutyczne Polpharma S.A., 83-200 Starogard Gdanski (PL)
(72) Inventor: PADUSZYNSKI, Piotr, 98-346 Skomlin (PL); MADANECKA, Anna, 83-200 Starogard Gdanski (PL)

(57) **Abstract**

The invention relates to a dry powder blend pharmaceutical composition comprising: a) a therapeutically effective amount of ramipril or a pharmaceutically acceptable salt thereof, or a hydrate thereof; b) a therapeutically effective amount of indapamide, or a hydrate thereof; c) microcrystalline cellulose; d) mannitol; and e) optionally one or more other pharmaceutically acceptable excipients. It also relates to an oral unit dosage form comprising the dry powder blend pharmaceutical composition and to a process for the preparation of the blend comprising dry mixing the components.

## Description

### Technical Field

The present invention relates to a dry powder blend pharmaceutical composition comprising ramipril and indapamide, and to an oral unit dosage form comprising them. It also relates to a process for the preparation of said dry powder blend pharmaceutical composition.

### Background Art

Ramipril (CAS Registry Number 87333-19-5) is an Angiotensin-Converting Enzyme (ACE) inhibitor useful for treating hypertension, heart failure, and diabetic kidney disease. It can also be used in patients over 55 years old to reduce the risk of having a heart attack, stroke or cardiovascular death in patients shown to be at high risk, such as some diabetics and patients with vascular diseases.

The chemical name of ramipril is (2S,3aS,6aS)-1-[(2S)-2-[[(2S)-1-ethoxy-1-oxo-4-phenylbutan-2-yl]amino]propanoyl]-3,3a,4,5,6,6*a*-hexahydro-2*H*-cyclopenta[b]pyrrole-2-carboxylic acid. It has the following chemical structure:

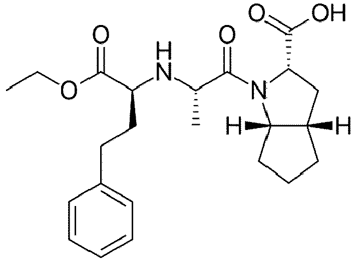

Indapamide (CAS Registry Number 26807-65-8) is a thiazide-like diuretic drug used in the treatment of hypertension, as well as decompensated heart failure. Its chemical name is 4-chloro-N-(2-methyl-2,3-dihydroindol-1-yl)-3-sulfamoylbenzamide. Indapamide has the following chemical structure:

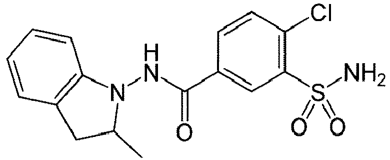

Fixed dose compositions comprising ACE inhibitors and diuretics have been successfully used in the prevention of cardiovascular complications in patients with hypertension. Within this context fixed dose combinations of ramipril and indapamide have been described in the prior art. For example, the Russian patent RU2618471 discloses an oral pharmaceutical composition containing ramipril and indapamide in which the active ingredients are both in micronized form.

This document explains that the oral use of ACE inhibitors such as ramipril is prevented by the poor dissolution when they are in the normal crystalline form, which leads to a reduction of the bioavailability of active ingredients. According to the inventors of RU2618471, adding additional auxiliary substances in the preparation of the solid dosage form, such as surfactants, disintegrants, etc., does not lead to the desired rate of the dissolution.

RU2618471 discloses that micronization (i.e., reduction of the particle size) of the active substances ramipril and indapamide to produce particles having an average size less than 50 microns, allows achieving the desired dissolution profile (at least 75% for 45 minutes in 0.1 N HCl at a temperature of 37 °C) for each of the active ingredients with a minimum number of auxiliary components.

It is also disclosed in this document that this technical result is achieved by a method of manufacture wherein the micronized active ingredients are screened and blended, later on mixed with excipients such as lactose and colloidal silicon dioxide, and then wet granulated. The obtained granules are dried, mixed with further excipients such as colloidal silicon dioxide and calcium stearate, and the final mixture is encapsulated into hard gelatine capsules.

The above process has the disadvantage that it involves wet granulation. Therefore, it includes at least an additional subsequent drying step since the granulated particles must be dried, and the drying process may require another equipment.

Therefore, there is still a need to find a new combined preparation containing ramipril and indapamide which while having a suitable dissolution profile can be prepared by a process that overcomes the drawbacks of the prior art.

### Summary of Invention

The inventors have found that when ramipril and indapamide are dry blended with specific amounts of mannitol and microcrystalline cellulose, a dry powder blend pharmaceutical composition can be obtained, which shows good blend uniformity. Besides, in the experiments carried out by the inventors it was observed that formulations according to the invention showed improved blend uniformity with respect to formulations which did not contain both mannitol and cellulose microcrystalline.

Further, as illustrated in the examples below it was found that capsules containing the dry powder blend pharmaceutical composition of the invention showed good content uniformity, and also good storage stability under different conditions of time, temperature and relative humidity. In this regard, formulations according to the invention showed improved lower total impurity profile with respect to a formulation which did not contain both mannitol and microcrystalline cellulose.

On the other hand, the oral dosage form of the invention shows a desired dissolution profile for both active ingredients such that at least 75% by weight of each active ingredient is dissolved within 15 minutes. In this regard, the appropriate blend and content uniformity of the dosage form reduce any possible variability of the dissolution profile among different finished dosage forms such as capsules.

Therefore, a first aspect of the invention relates to a dry powder blend pharmaceutical composition comprising:
a) a therapeutically effective amount of ramipril or a pharmaceutically acceptable salt thereof, or a hydrate thereof;
b) a therapeutically effective amount of indapamide or a hydrate thereof;
c) microcrystalline cellulose;
d) mannitol; and
e) optionally one or more other pharmaceutically acceptable excipients.

As mentioned above, the dry powder blend pharmaceutical composition can be conveniently administered in the form of capsules, in particular hard gelatine capsules. Thus, a second aspect of the invention refers to an oral unit dosage form comprising the dry powder blend pharmaceutical composition as defined herein.

The dry powder blend pharmaceutical composition of the invention can be prepared by simply dry blending the active ingredients and excipients without the need of using solvents (as is the case of wet granulation) which have to be removed in subsequent steps. This makes the process much simpler. Besides, the fact that the process includes less steps and conditions that may cause degradation of the active ingredients has a direct impact on the stability of the composition and the impurity profile. In fact, this has been confirmed in the examples below: a formulation according of the invention showed fewer total impurities (at 6 months, 40°C, 75% RH) than a formulation prepared by wet granulation. In summary, the process for the preparation of the dry powder blend pharmaceutical composition of the invention is robust, reproducible, cost-effective, and easy to scale-up at an industrial level.

Thus, a third aspect of the invention relates to a process for the preparation of the dry powder blend pharmaceutical composition as previously defined, which comprises dry mixing ramipril or a pharmaceutically acceptable salt thereof, or a hydrate thereof; indapamide a hydrate thereof; microcrystalline cellulose; mannitol; and optionally one or more other pharmaceutically acceptable excipients.

Another aspect of the invention relates to the dry powder blend pharmaceutical composition as previously defined or oral unit dosage form comprising it for use in the treatment of hypertension. Therefore, this aspect relates to the use of ramipril, or a pharmaceutically acceptable salt thereof, or a hydrate thereof, and indapamide, or a hydrate thereof, for the manufacture of the dry powder blend pharmaceutical composition as previously defined or oral unit dosage form comprising it for the treatment of hypertension. Alternatively, this aspect may also be formulated as a method for the treatment of hypertension in a mammal, including a human, the method comprising administering to said mammal the previously defined dry powder blend pharmaceutical composition or oral unit dosage form comprising it.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims.

The term "about" or "around" as used herein refers to a range of values ± 10% of a specified value. For example, the expression "about 10" or "around 10" includes ± 10% of 10, i.e. from 9 to 11.

For the purposes of the present invention, any ranges given include both the lower and the upper endpoints of the range. Ranges and values given, such as temperatures, times, and the like, should be considered approximate, unless specifically stated.

The terms "percentage (%) by weight" or "percentage (%) w/w" have the same meaning and are used interchangeable. This term refers to the percentage of a component in relation to the total weight.

The term "dry powder blend pharmaceutical composition" is used herein to refer to a pharmaceutical composition, particularly a solid dosage form, which has been prepared by dry blending or dry mixing. The terms "dry blending" or "dry mixing" are used interchangeably and refer to the process of homogenously mixing the components of the pharmaceutical composition, wherein the ingredients are added in a dry powder form. Such process is therefore carried out in the absence of any liquids or solvents, such as water, ethanol or combinations thereof.

The terms "micronized" or "milled" as used interchangeably to mean that the active ingredient has been subjected to size reduction by any means in such a way that the final average particle size is particularly lower than 50 µm.

The expression "therapeutically effective amount" as used herein, refers to the amount of ramipril and indapamide that, when administered, is sufficient to prevent development of, or alleviate to some extent, one or more of the symptoms of the disease to which it is addressed. The specific dose of ramipril and indapamide to obtain a therapeutic benefit may vary depending on the particular circumstances of the individual patient including, among others, the size, weight, age and sex of the patient, and the nature and stage of the disease. The appropriate therapeutically effective amount can readily be determined by a skilled in the art according to the type of disease to which it is addressed.

The expression "pharmaceutically acceptable excipients" as used herein refers to pharmaceutically acceptable materials, compositions or vehicles. Each component must be pharmaceutically acceptable in the sense of being compatible with the other ingredients of the pharmaceutical composition. They must also be suitable for use in contact with the tissue or organ of humans and animals without excessive toxicity, irritation, allergic response, immunogenicity or other problems or complications commensurate with a reasonable benefit/risk ratio. The appropriate excipients and their amounts can readily be determined by a skilled in the art according to the type of formulation being prepared.

The term "oral unit dosage form" as used herein refers to a drug product which has undergone all stages of manufacture and is administered to a patient as a medication by oral administration, i.e., by ingesting the drug by swallowing or chewing, preferably, by swallowing. The oral unit dosage form according to the invention comprises the dry powder blend pharmaceutical composition as defined herein. As an example the oral unit dosage form may also be a capsule. The oral unit dosage form may also comprise a packaging such as sachets, blisters or bottles.

The term "blend uniformity" as used herein, refers to the homogeneity of the dry powder blend composed of the active ingredients and the excipients before converting the blend into the finished oral unit dosage form. Thus, for assessing the blend uniformity of capsules for example the mixture of ingredients prior to filling it in the capsule is evaluated.

The term "content uniformity" as used herein refers to the homogeneity of the drug content among individual units of finished oral unit dosage form, e.g., capsules. Thus, for assessing the content uniformity different capsules for example are assayed.

The term "hard capsule" refers to a capsule in which a content (capsule fill) is filled into a produced capsule film. Typically, hard capsules are made up of two cylindrical halves, one of them being wider in diameter but shorter in length (cap), and the other one being narrower in diameter but longer in length (body).

The dry powder blend pharmaceutical composition of the invention comprises ramipril or a pharmaceutically acceptable salt thereof, or a hydrate thereof, and indapamide, or a hydrate thereof.

There is no limitation on the type of salt of ramipril that can be used, provided that they are pharmaceutically acceptable when used for therapeutic purposes. The term "pharmaceutically or veterinary acceptable salts" embraces salts commonly used to form alkali metal salts and to form addition salts of free bases. Non-limiting examples of ramipril salts include salts with bases such as alkali metal or alkaline earth metal salts, e.g., sodium, potassium, calcium or magnesium salts, or salts with ammonia or an organic amine, such as morpholine, pyrrolidine, arginine or a lower alkylamine such as ethylenediamine, N-methyl-glucamine (meglumine) or t-butylamine.

The preparation of pharmaceutically acceptable salts may be carried out by methods well-known in the art. Generally, such salts may be prepared by reacting ramipril with a stoichiometric amount of the appropriate pharmaceutically acceptable base in an appropriate solvent such as water, an organic solvent, or a mixture thereof. The obtained salts may differ in some properties with respect to ramipril in free acid form, but they are considered equivalent for the purposes of the present invention.

According to one embodiment, optionally in combination with one or more features of the various embodiments described above or below, ramipril is present in the dry powder blend pharmaceutical composition as ramipril in free acid form (i.e., not in the form of a salt).

The active ingredients of the composition of the invention may be in crystalline form either as free solvation compounds or as solvates, in particular hydrates and it is intended that both forms are within the scope of the present invention. Methods of solvation are generally known within the art. In general, the solvated (i.e., hydrated) forms with pharmaceutically or veterinary acceptable solvents such as water are equivalent to the unsolvated (i.e. non-hydrated) form for the purposes of the invention.

In one embodiment, optionally in combination with one or more features of the various embodiments described above or below, ramipril is present in the dry powder blend pharmaceutical composition as ramipril in non-solvated (non-hydrated) form, more particularly, in free acid non-solvated (non-hydrated) form.

In another embodiment, optionally in combination with one or more features of the various embodiments described above or below, indapamide is present in the dry powder blend pharmaceutical composition as indapamide hemihydrate.

In another embodiment, optionally in combination with one or more features of the various embodiments described above or below, the dry blend pharmaceutical composition of the invention comprises ramipril, or a pharmaceutically acceptable salt thereof, or a hydrate thereof, in an amount from 1 to 10%, more particularly from 2 to 8%, even more particularly from 3 to 6%, and even more particularly from 4 to 5%, by weight with respect to the total weight of the composition.

In another embodiment, optionally in combination with one or more features of the various embodiments described above or below, the dry blend pharmaceutical composition of the invention comprises indapamide, or a hydrate thereof, in an amount from 0.5 to 3%, more particularly from 0.5 to 2%, even more particularly from 0.5 to 1.5%, and even more particularly from 0.6 to 1.3%, by weight with respect to the total weight of the composition.

In the compositions of the invention the active ingredients ramipril and indapamide may be in micronized or unmicronized form.

In one embodiment, optionally in combination with one or more features of the various embodiments described above or below, the dry powder blend pharmaceutical composition of the invention comprises ramipril, or a pharmaceutically acceptable salt thereof, or a hydrate thereof, in an unmicronized form.

In another embodiment, optionally in combination with one or more features of the various embodiments described above or below, the dry powder blend pharmaceutical composition of the invention comprises indapamide, or a hydrate thereof, in an unmicronized form.

According to another embodiment, optionally in combination with one or more features of the various embodiments described above or below, the dry powder blend pharmaceutical composition of the invention comprises ramipril, or a pharmaceutically acceptable salt thereof, or a hydrate thereof, in micronized form, wherein the average particle size of ramipril is equal or lower than 50 µm, more particularly equal or lower than 45 µm, or equal or lower than 40 µm, or equal or lower than 35 µm, or equal or lower than 30 µm, or equal or lower than 25 µm.

In another embodiment, optionally in combination with one or more features of the various embodiments described above or below, the dry powder blend pharmaceutical composition of the invention comprises indapamide, or a hydrate thereof, in micronized form, wherein the average particle size of indapamide is equal or lower than 50 µm, more particularly 45 µm, or equal or lower than 40 µm, or equal or lower than 35 µm, or equal or lower than 30 µm, or equal or lower than 25 µm, or equal or lower than 20 µm, or equal or lower than 15 µm.

In another embodiment, optionally in combination with one or more features of the various embodiments described above or below, in the dry powder blend pharmaceutical composition of the invention both ramipril and indapamide are in micronized form, wherein the average particle size of ramipril and indapamide is independently equal or lower than 50 µm, more particularly 45 µm, or equal or lower than 40 µm, or equal or lower than 35 µm, or equal or lower than 30 µm, or equal or lower than 25 µm, or equal or lower than 20 µm, or equal or lower than 15 µm.

The average particle size of the active ingredients may be measured by methods well-known in the art such as light microscope and scanning electron microscope, sieve analysis or laser diffraction.

The dry blend compositions of the invention further comprise mannitol and microcrystalline cellulose.

Mannitol (CAS Registry number 69-65-8) is a type of sugar alcohol which may be used as excipient for a variety of purposes in pharmaceutical formulations. Mannitol has the following formula:

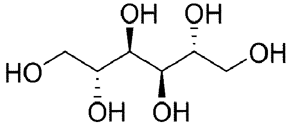

In one embodiment, optionally in combination with one or more features of the various embodiments described above or below, mannitol is present in the dry powder blend pharmaceutical composition in an amount from 5 to 80%, more particularly from 10 to 70%, or from 20 to 60%, or from 30 to 50%, or about 40% by weight with respect to the total weight of the composition.

Microcrystalline cellulose (MCC) is a partially depolymerized cellulose is composed of glucose units connected by a 1-4 beta glycosidic bond. It is obtained as a pulp from fibrous plant material. Typically, the degree of polymerization of microcrystalline cellulose is less than 400, more particularly from 100 to 300, and the carbohydrate content is generally over 97%, calculated as cellulose on an anhydrous basis. Microcrystalline cellulose is mostly used as a binder or diluent in oral pharmaceutical formulations.

It is commercially available in a range of particle sizes (median particle diameter (D50) from 50 to 200 µm) and moisture contents (typically less than 5% by weight), for example as: Avicel PH-101 (particle size: 50 µm, moisture content: 3.0 - 5.0%), Avicel PH-102 (particle size: 100 µm, moisture content: 3.0 - 5.0%), Avicel PH-103 (particle size: 50 µm, moisture content: NMT 3.0%), Avicel PH-105 (particle size: 20 µm, moisture content: NMT 5.0%), Avicel PH-112 (particle size: 100 µm, moisture content: NMT 1.5%), Avicel PH-113 (particle size: 50 µm, moisture content: NMT 2.0%), Avicel PH-200 (particle size: 200 µm, moisture content: 2.0 - 5.0%), Avicel PH-200 LM (particle size: 200 µm, moisture content: NMT 1.5%), Avicel PH-301 (particle size: 50 µm, moisture content: 3.0 - 5.0%), Avicel PH-302 (particle size: 100 µm, moisture content: 3.0 - 5.0%) or Vivapur 12 (particle size: 230 µm, moisture content: 3.0 - 5.0%).

The particle size may be measured by laser diffraction, for example, a Malvern laser diffraction unit, Malvern Mastersizer 3000 particle size analyzer, from Malvern Instruments Ltd., Malvern, England may be employed. The term D(50) is a value expressing the median particle diameter and means that 50% of the particles have a particle size below this value.

According to one embodiment, optionally in combination with one or more features of the various embodiments described above or below, the microcrystalline cellulose present in the dry powder blend pharmaceutical composition of the invention has a median particle diameter (D50) from 20 to 500 µm, more particularly from 35 to 450 µm, or from 50 to 400 µm, or from 100 to 300 µm, and even more particularly about 200 µm.

In another embodiment, optionally in combination with one or more features of the various embodiments described above or below, the microcrystalline cellulose present in the dry powder blend pharmaceutical composition of the invention has moisture content from 0 to 10%, more particularly from 3 to 7%, and even more particularly about 3 to 4% by weight with respect to the total microcrystalline cellulose weight.

In another embodiment, optionally in combination with one or more features of the various embodiments described above or below, microcrystalline cellulose is present in the dry powder blend pharmaceutical composition of the invention in an amount from 5 to 80%, or from 10 to 70%, or from 20 to 60%, or from 30 to 55%, or more particularly about 49% or 53%, by weight with respect to the total weight of the composition.

In another embodiment, optionally in combination with one or more features of the various embodiments described above or below, the dry powder blend pharmaceutical composition of the invention comprises:
a) ramipril, or a pharmaceutically acceptable salt thereof, or a hydrate thereof, in an amount from 1 to 10%, more particularly from 2 to 8%, even more particularly from 3 to 6%, and even more particularly from 4 to 5%;
b) indapamide, or a hydrate thereof, in an amount from 0.5 to 3%, more particularly from 0.5 to 2%, even more particularly from 0.5 to 1.5%, and even more particularly from 0.6 to 1.3%;
c) microcrystalline cellulose in an amount from 5 to 80%, or from 10 to 70%, or from 20 to 60%, or from 30 to 55%, or more particularly about 49% or 53%;
d) mannitol in an amount from 5 to 80%, more particularly from 10 to 70%, or from 20 to 60%, or from 30 to 50%, or about 40% by weight with respect to the total weight of the composition; and
e) optionally one or more other pharmaceutically acceptable excipients;
wherein the % are expressed in weight with respect to the total weight of the composition, provided that the sum of the amounts of the components is less than or equal to 100%. o

In another embodiment, optionally in combination with one or more features of the various embodiments described above or below, the weight ratio between microcrystalline cellulose and mannitol is from 5:0.5 to 0.5:5, more particularly from 4:1 to 1:4 or from 1:1 to 4:3, and even more particularly about 4:3.

An advantage of the dry powder blend pharmaceutical composition of the invention is that it is very simple in the sense that only few excipients are needed to obtain all desired properties of stability, blend and content uniformity, and dissolution profile. This is favourable because ramipril is very unstable and has a lot of incompatibilities with many chemicals.

In some embodiments, the dry powder blend pharmaceutical composition of the invention may comprise one or more other pharmaceutically acceptable excipients. Examples of appropriate additional pharmaceutically acceptable excipients include without limitation lubricants, disintegrants, binders, diluents, glidants or combinations thereof.

The term "lubricant" as used herein refers to a material which is able to decrease adhesion of a powder to punches and friction between particles. Non-limiting examples of lubricants include magnesium stearate, calcium stearate, zinc stearate, high molecular weight polyethylene glycol, stearic acid, sodium stearyl fumarate, or combinations thereof.

The term "disintegrant" as used herein refers to a material which facilitates the release of the active ingredients of a pharmaceutical composition on contact with moisture. Non-limiting examples of disintegrants include, without limitation, natural, modified or maize starch, pregelatinized starch, crospovidone, croscarmellose sodium, sodium starch glycolate, low-substituted hydroxypropyl cellulose, or combinations thereof.

The term "binder" refers to a material which imparts cohesiveness to powdered materials improving free-flowing qualities. Non-limiting examples of binders include starch, pregelatinized starch, sodium carboxymethylcellulose, polyvinylpyrrolidone (povidone), copolymers of vinylpyrrolidone with other vinylderivatives (copovidone), hydroxypropylmethylcellulose, methylcellulose, and hydroxypropylcellulose, polyethylene glycol, hydrolyzed gelatin, natural gums, polyvinyl alcohol or combinations thereof.

The term "glidant" refers to a material which improves the flow characteristics of powder mixtures in the dry state. Non-limiting examples of glidants are colloidal silicon dioxide, talc, or combinations thereof.

The term "diluent" refers to a material which are added to the bulk volume of the active ingredients making up the solid composition. Non-limiting examples of diluents are lactose, calcium carbonate, dibasic calcium phosphate, dextrose, fructose, isomaltose, magnesium carbonate, magnesium oxide, maltose, sorbitol, sodium alginate, sucrose, xylitol, or combinations thereof.

According to one embodiment, optionally in combination with one or more features of the various embodiments described above or below, the dry powder blend pharmaceutical composition of the invention further comprises one or more other pharmaceutically acceptable excipients selected from the group consisting of lubricants, disintegrants, binders, diluents, glidants, and combinations thereof, more particularly, selected from the group consisting of lubricants, disintegrants, and a combination thereof.

In another embodiment, optionally in combination with one or more features of the various embodiments described above or below, the dry powder blend pharmaceutical composition of the invention further comprises a lubricant, such as magnesium stearate, more particularly in an amount from 0.1 to 10%, more particularly from 0.5 to 5%, by weight with respect to the total weight of the composition.

In another embodiment, optionally in combination with one or more features of the various embodiments described above or below, the dry powder blend pharmaceutical composition of the invention further comprises a disintegrant, such as crospovidone, more particularly in an amount from 1 to 15%, more particularly from 2 to 6%, by weight with respect to the total weight of the composition.

In another embodiment, optionally in combination with one or more features of the various embodiments described above or below, the dry powder blend pharmaceutical composition of the invention comprises or consists of:
a) from 1 to 10% of ramipril or a pharmaceutically acceptable salt thereof, or a hydrate thereof;
b) from 0.5 to 3% of indapamide, or a hydrate thereof;
c) from 5 to 80% of microcrystalline cellulose;
d) from 5 to 80% of mannitol; and
e) a lubricant, particularly magnesium stearate, in an amount from 0.1 to 10%;
wherein the % are expressed in weight with respect to the total weight of the composition, provided that the sum of the amounts of the components is less than or equal to 100%.

In another embodiment, optionally in combination with one or more features of the various embodiments described above or below, the dry powder blend pharmaceutical composition of the invention comprises or consists of:
a) from 1 to 10% of ramipril or a pharmaceutically acceptable salt thereof, or a hydrate thereof;
b) from 0.5 to 3% of indapamide, or a hydrate thereof;
c) from 5 to 80% of microcrystalline cellulose;
d) from 5 to 80% of mannitol; and
e) a lubricant, particularly magnesium stearate, in an amount from 0.1 to 10%; and
f) a disintegrant, particularly crospovidone, in an amount from 1 to 15%;
wherein the % are expressed in weight with respect to the total weight of the composition, provided that the sum of the amounts of the components is less than or equal to 100%.

It is well known that in some patients the presence of lactose in some patients may cause lactose intolerance discomfort. Thus, in one embodiment, optionally in combination with one or more features of the various embodiments described above or below, in the dry powder blend pharmaceutical composition of the invention lactose is absent.

In another embodiment, optionally in combination with one or more features of the various embodiments described above or below, in the dry powder blend pharmaceutical composition of the invention colloidal silica is absent.

The dry blend compositions of the invention are typically administered by the oral route. Thus, in one embodiment, optionally in combination with one or more features of the various embodiments described above or below, the dry powder blend pharmaceutical composition of the invention is an oral composition.

It also forms of the invention an oral unit dosage form comprising the dry powder blend pharmaceutical composition as defined herein. In one embodiment, optionally in combination with one or more features of the various embodiments described above or below, the oral unit dosage form of the invention is a capsule, more particularly a hard capsule, and even more particularly a hard gelatine capsule.

The capsule size may range from size 0 to size 5, more particularly a capsule of size 0, size 1, size 2, size 3, size 4 or size 5 can be used. In one embodiment, optionally in combination with one or more features of the various embodiments described above or below, the capsule has size 1.

The hard capsule can be made of a substance including an animal protein such as gelatine, and plant polysaccharide or their derivatives such as hydroxypropyl methylcellulose (hypromellose, HPMC), pullulan, carrageenans or mixtures thereof. In addition to the above-mentioned components, further excipients can also be added to the capsules. Non-limiting examples of such excipients include opacifying agents such as titanium dioxide, and colorants such as iron oxide yellow, iron oxide black, iron oxide red or plant colorants such as flavonoids.

According to one embodiment, optionally in combination with one or more features of the various embodiments described above or below, the hard capsule is a gelatine hard capsule. Hard capsules made with gelatin, generally include about 80% by weight of gelatin and 15% by weight of water.

In another embodiment, optionally in combination with one or more features of the various embodiments described above or below, the hard capsule is made of gelatine and an opacifying agent such as titanium dioxide.

In another embodiment, optionally in combination with one or more features of the various embodiments described above or below, the hard capsule is made of gelatine and is devoid of titanium dioxide. More particularly, hard capsule is made of gelatine and from 1 to 4% w/w of iron oxide, i.e., red or yellow one or a mixture thereof.

The oral unit dosage form of the invention, particularly in the form of hard capsules, can be conditioned in a suitable packaging. Typically, primary, and secondary packaging will be used. A skilled in the art will easily select the appropriate type of packaging depending on the type of formulation being prepared.

In one embodiment, optionally in combination with one or more features of the various embodiments described above or below, the dry powder blend pharmaceutical composition of the invention is packaged in blisters.

In another embodiment, optionally in combination with one or more features of the various embodiments described above or below, the dry powder blend pharmaceutical composition of the invention is primary packaged in blisters and secondary packaged in an outer carton.

Non-limiting examples of materials used for assembling the blisters include PVC (polyvinylchloride), aluminium (Al), oriented polyamide (OPA, also known as nylon), PVdC (polyvinylidene chloride), polyethylene (PE) including high-density polyethylene (HDPE) and low-density polyethylene (LDPE)), polyethylene terephthalate (PET), or a combination thereof.

According to one embodiment, optionally in combination with one or more features of the various embodiments described above or below, the blisters are AI/OPA/AI/PVC foil blisters.

In one embodiment, optionally in combination with one or more features of the various embodiments described above or below, the oral unit dosage form, particularly in the form of a hard capsule, comprises ramipril, or a pharmaceutically acceptable salt thereof, or a hydrate thereof, in an amount from 2 to 20 mg, more particularly, from 5 to 15 mg, and even more particularly about 10 mg, based on ramipril free acid non-solvated form.

In another embodiment, optionally in combination with one or more features of the various embodiments described above or below, the oral unit dosage form, particularly in the form of a hard capsule, comprises indapamide, or a hydrate thereof, in an amount from 0.75 to 5 mg, more particularly, from 1.0 to 4.0 mg, and even more particularly about 1.25 or about 2.5 mg, based on indapamide in non-solvated form.

As mentioned above, the oral unit dosage form of the present invention shows and adequate dissolution profile for both active ingredients. In the present invention, the measurement of the dissolution profile is performed in conditions: basket type apparatus (USP dissolution Apparatus 1), at 100 rpm (revolution per minute), the medium volume 900 mL in 37°C ± 0.5 °C) or paddle-type apparatus (USP dissolution apparatus 2, 75 rpm (rotation per minute), 900 mL of dissolution media in 37°C ± 0.5 °C). Medium QC is 0.1 N HCl.

In one embodiment, optionally in combination with one or more features of the various embodiments described above or below, the oral unit dosage form of the invention exhibits a dissolution profile according to which at least 75%, more particularly at least 80%, and even more particularly at least 85%, by weight of ramipril, or a pharmaceutically acceptable salt thereof, or a hydrate thereof, is dissolved in a 0.1M HCl medium 15 minutes.

In one embodiment, optionally in combination with one or more features of the various embodiments described above or below, the oral unit dosage form of the invention exhibits a dissolution profile according to which at least 75%, more particularly at least 80%, and even more particularly at least 85%, by weight of indapamide or a hydrate thereof, is dissolved in a 0.1M HCl medium within 15 minutes.

It also forms part of the invention a process for the preparation of the dry powder blend pharmaceutical composition as previously defined, which comprises dry mixing ramipril, or a pharmaceutically acceptable salt thereof, or a hydrate thereof; indapamide, or a hydrate thereof; microcrystalline cellulose; mannitol; and optionally one or more other pharmaceutically acceptable excipients.

With the aim of preparing an oral unit dosage form, the process further comprises providing the oral unit dosage form. For example, when the finished oral unit dosage form is a hard capsule, the process comprises filling the capsule. Methods for preparing hard capsules are well-known in the art. Furthermore, hard capsules such as hard gelatin capsules are also commercially available. Thus, in one embodiment, optionally in combination with one or more features of the various embodiments described above or below, the process comprises the following steps:
a) dry mixing ramipril or a pharmaceutically acceptable salt thereof, or a hydrate thereof; indapamide or a hydrate thereof; microcrystalline cellulose; mannitol; and optionally one or more other pharmaceutically acceptable excipients; and
b1) filling the hard capsule with the mixture obtained in step a); and
b2) optionally sealing the hard capsule.

Step a) comprising the dry mixing of all the components of the formulation can be carried out in different steps.

According to one embodiment, optionally in combination with one or more features of the various embodiments described above or below, step a) of the process comprises the following steps:
i) dry mixing ramipril, or a pharmaceutically acceptable salt thereof, or a hydrate thereof, with part 1 of the microcrystalline cellulose;
ii) dry mixing indapamide, or a hydrate thereof with part 2 of the microcrystalline cellulose;
iii) dry mixing the mixture of step i), the mixture of step ii) and part 3 of the microcrystalline cellulose:
iv) dry mixing the mixture of step iii), mannitol, part 4 of the microcrystalline cellulose, and optionally one or more other pharmaceutically acceptable excipients except the lubricant (if present); and
v) dry mixing the mixture of step iv) and the lubricant (if present);
wherein steps i) and ii) may be carried out in any order, and part 1 of the microcrystalline cellulose represents from 2 to 25% of the total microcrystalline cellulose present in the composition, part 2 of the microcrystalline cellulose represents from 2 to 25% of the total microcrystalline cellulose present in the composition, part 3 of the microcrystalline cellulose represents from 2 to 25% of the total microcrystalline cellulose present in the composition, and part 4 of the microcrystalline cellulose represents from 25 to 94% of the total microcrystalline cellulose present in the composition. In one particular embodiment, parts 1, 2 and 3 of the microcrystalline cellulose represent each independently from 5 to 25% of the total microcrystalline cellulose present in the composition, and part 4 of the microcrystalline cellulose represents from 25 to 75%. In another particular embodiment, parts 1, 2 and 3 of the microcrystalline cellulose represent each independently from 2 to 10% of the total microcrystalline cellulose present in the composition, and part 4 of the microcrystalline cellulose represents from 70 to 94%.

Any of the above mixtures can be carried out for example at 12 rpm for a time period comprised from 5 to 75 minutes.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Materials

Ramipril (particle size D(0.9) 25µm), indapamide (particle size D(0.9) 15 µm), microcrystalline cellulose (JRS Pharma GMBH & Co. KG, particle size: D(0.5) 230 µm, moisture content: less than 5%), mannitol (Roquette), crospovidone (JH Nanhang Life Sciences Co., Ltd.), magnesium stearate (Nitika Pharmaceutical Specialities PVT. LTD.), pregelatinized starch (Colorcon INC.), gelatin capsules (Capsugel Belgium NV, bovine hard gelatin capsules, size no 1).

### General procedure for manufacturing hard capsules according to the invention

Mixing and encapsulation:
1. Ramipril and microcrystalline cellulose (part 1: from 2 to 10% of the whole cellulose amount) were weighed and mixed in polyethylene bag or handling tank/bin (trituration of ramipril).
2. Indapamide and microcrystalline cellulose (part 2: from 2 to 10% of the whole cellulose amount) were weighed and mixed in polyethylene bag or handling tank/bin (trituration of indapamide).
3. Microcrystalline cellulose (part 3: from 2 to 10% of the whole cellulose amount and part 4: from 70 to 94% of the whole cellulose amount), mannitol, crospovidone (if present) and magnesium stearate were weighed.
4. Trituration of ramipril and trituration of indapamide were screened to the bin together with microcrystalline cellulose part 3.
5. Screened materials were mixed for 10 minutes at speed 12 rpm.
6. Microcrystalline cellulose (part 4), mannitol, crospovidone (if present), and pregelatinized starch (if present) were screened and added to the mixture with ramipril and indapamide.
7. The whole mixture was mixed for 30 minutes at speed 12 rpm.
8. Magnesium stearate (if present) was screened, added to the mixture and mixed for 7 minutes at speed 12 rpm.
9. The capsule mass was encapsulated on encapsulation machine (capsule no 1).

Packing:
10. Ramipril/lndapamide hard capsules were packed in AL//OPA/AI/PVC foil blisters.

The compositions described below were prepared following the general procedure mentioned above.

### Capsules filled with composition 1 (Capsules 1)

| **Ingredient** | **mg/capsule** | **% w/w** |
|---|---|---|
| Ramipril | 10.00 | 4.88 |
| Indapamide hemihydrate | 2.56 | 1.25 |
| Microcrystalline cellulose | 108.20 | 52.79 |
| Mannitol | 82.14 | 40.07 |
| Magnesium stearate | 2.10 | 1.01 |
| **Total (capsule fill)** | **205.00** | **100** |
| Bovine gelatin | 74.48 | |
| Titanium dioxide | 1.52 | |
| **Capsule weight (total)** | **281.00** | |

This formulation showed satisfactory flowability; flow rate (diameter 15 mm) = 11.4 s (average: 11.1 s).

### Capsules filled with composition 2 (Capsules 2)

| **Ingredient** | **mg/capsule** | **% w/w** |
|---|---|---|
| Ramipril | 10.00 | 4.88 |
| Indapamide hemihydrate | 2.56 | 1.25 |
| Microcrystalline cellulose | 100.00 | 48.78 |
| Crospovidone | 8.20 | 4.00 |
| Mannitol | 82.14 | 40.07 |
| Magnesium stearate | 2.10 | 1.01 |
| **Total (capsule fill)** | **205.00** | **100** |
| Bovine gelatin | 74.48 | |
| Titanium dioxide | 1.52 | |
| **Capsule weight (total)** | **281.00** | |

This formulation showed satisfactory flowability; flow rate (diameter 15 mm) = 15.8-16.7 s (average: 16.4 s).

### Capsules filled with comparative composition 1 (Comparative capsules 1)

| **Ingredient** | **mg/capsule** | **% w/w** |
|---|---|---|
| Ramipril | 10.00 | 4.88 |
| Indapamide hemihydrate | 2.56 | 1.25 |
| Mannitol | 190.34 | 92.85 |
| Magnesium stearate | 2.10 | 1.02 |
| **Total (capsule fill)** | **205.00** | **100** |
| Bovine gelatin | 74.48 | |
| Titanium dioxide | 1.52 | |
| **Capsule weight (total)** | **281** | |

### Capsules filled with comparative composition 2 (Comparative capsules 2)

| **Ingredient** | **mg/capsule** | **% w/w** |
|---|---|---|
| Ramipril | 10.00 | 4.88 |
| Indapamide hemihydrate | 1.28 | 0.62 |
| Microcrystalline cellulose | 109.48 | 53.405 |
| Pregelatinized starch | 82.14 | 40.07 |
| Magnesium stearate | 2.10 | 1.02 |
| **Total (capsule fill)** | **205.00** | **100** |
| Bovine gelatin | 74.48 | |
| Titanium dioxide | 1.52 | |
| **Capsule weight (total)** | **281** | |

This formulation showed very poor flowability; the flow rate (diameter 15 mm) was 11.4-50.0 s (average: 30 s).

### General procedure for manufacturing hard capsules filled with a composition made by wet granulation

1. Screening: sieve 1.2 mm (ramipril, indapamide, lactose)
2. Granulation process:
   a. Blending: chopper speed - 1500 rpm, impeller speed - 500, blending time: 180 s.
   b. wet granulation:
      - binder addition phase: chopper speed - 1500 rpm, impeller speed - 500, blending time: 187 s.
      - granulation phase: chopper speed - 2000 rpm, impeller speed - 500, blending time: 60 s.
3. Drying:
   - inlet air temperature - 36/37 °C
   - outlet air temperature - 23/26 °C
   - product temperature - 28/31 °C
   - drying air volume - 30 - 50 m³/h
   - drying time: 15 min
   - granulate LOD (end of process): 0.43%
4. Mixing: Add colloidal silica sieved through a 1 mm sieve to the dried granulate - mix in the bin for 10 min 12 rpm then add magnesium stearate - mix 7 min 12 rpm
5. Encapsulation

The composition described below was prepared following the general procedure mentioned above.

### Capsules filled with comparative composition 3 (Comparative capsules 3)

| **Intragranular components** | | **mg/caps.** | **% w/w** |
|---|---|---|---|
| 1 | Ramipril | 10.00 | 6.45 |
| 2 | Indapamide | 2.56 | 1.65 |
| 3 | Lactose monohydrate (Flowlac 100) | 139.50 | 89.97 |

| **Extragranular components** | | | |
|---|---|---|---|
| 4 | Silica colloidal anhydrous (Aerosil R200) | 1.50 | 0.97 |
| 5 | Magnesium Stearate | 1.50 | 0.97 |
| **Total (capsule fill)** | | **155.06** | **100.00** |
| Bovine gelatin | | 74.48 | |
| Titanium dioxide | | 1.52 | |
| **Capsule weight (total)** | | **231.06** | |

### Blend uniformity

Blend uniformity was measured by taking samples of the capsule mass or granulate from different parts of the bin (sampling points 1-10) in amount of approx. 0.2 - 0.6 g with proper sampler. Samples were taken from the top of the bin charge, the center, and the bottom by pouring from the sampler to glass bottles (the samples were taken for tests and spared samples in the amount of 10 x 2 [full scale] or 5 x 2 [laboratory scale]). In the following table, the blend uniformity of the compositions of the invention is shown (results correspond to % of the active substances in relation to dose (100%)):

| Sampling point | **Composition 1** | | **Composition 2** | | **Comparative composition 1** | | **Comparative composition 2** | |
|---|---|---|---|---|---|---|---|---|
| | Ramipril | Indapamide | Ramipril | Indapamide | Ramipril | Indapamide | Ramipril | Indapamide |
| 1 | 93.8 | 90.9 | 96.6 | 92.5 | 98.6 | 98.1 | 100.4 | 100.1 |
| 2 | 95.1 | 91.4 | 96.3 | 92.1 | 98.8 | 97.4 | 98.6 | 97.0 |
| 3 | 95.9 | 92.3 | 96.9 | 92.1 | 99.1 | 97.9 | 98.6 | 98.8 |
| 4 | 95.5 | 92.9 | 96.4 | 92.3 | 110.5 | 108.4 | 97.3 | 98.5 |
| 5 | 97.0 | 93.5 | 96.8 | 92.2 | 137.2 | 135.8 | 99.5 | 99.6 |
| 6 | 95.6 | 92.0 | 96.3 | 91.6 | --- | --- | 113.8 | 99.1 |
| 7 | 96.6 | 92.0 | 96.9 | 92.3 | --- | --- | 95.1 | 98.8 |
| 8 | 96.1 | 92.9 | 97.0 | 91.9 | --- | --- | 96.3 | 98.3 |
| 9 | 94.5 | 92.8 | 95.4 | 91.2 | --- | --- | 95.3 | 98.8 |
| 10 | 96.3 | 92.6 | 96.9 | 92.5 | --- | --- | 97.6 | 98.5 |
| AV | 95.6 | 92.3 | 96.5 | 92.1 | 108.9 | 107.5 | 98.2 | 98.8 |
| RSD | 1.0 | 0.8 | 0.5 | 0.4 | 15.3 | 15.3 | 5.8 | 0.8 |

| | | | | | | | | |
|---|---|---|---|---|---|---|---|---|
| * Acceptance limits: 90-110% of labelled API content, RSD NMT: 5% AV: average, RSD: Relative Standard Deviation, NMT: not more than. | | | | | | | | |

As can be seen the compositions of the invention are characterized with good blend uniformity, while the comparative composition showed less uniformity.

### Content uniformity (finished dosage form)

The test for content uniformity of preparations presented in dosage units is based on the assay of the individual contents of active ingredients of a number of dosage units (capsules) to determine whether the individual contents are within the limits set. The test for content uniformity was performed according to European Pharmacopoeia 7.0, 2.9.40. Uniformity of dosage units.

In the following tables, the blend uniformity of the capsules filled with compositions of the invention is shown (results correspond to % of active ingredient with respect to the nominal dose (100%)). The test was performed on 9 capsules taken at different steps of encapsulation:

| **Capsules 1** | | | | | | |
|---|---|---|---|---|---|---|
| Sampling point | Start of encaps. | Middle of encaps. | End of encaps. | Start of encaps. | Middle of encaps. | End of encaps. |
| | Ramipril | | | Indapamide | | |
| 1 | 97.4 | 101.6 | 98.4 | 95.2 | 98.0 | 96.3 |
| 2 | 100.0 | 100.0 | 99.7 | 97.8 | 97.3 | 98.3 |
| 3 | 99.9 | 99.6 | 100.7 | 97.1 | 96.4 | 96.1 |
| Av | 99.1 | 100.4 | 99.6 | 96.7 | 97.2 | 96.9 |
| RSD | 1.5 | 1.1 | 1.2 | 1.4 | 0.8 | 1.3 |

| **Capsules 2** | | | | | | |
|---|---|---|---|---|---|---|
| Sampling point | Start of encaps. | Middle of encaps. | End of encaps. | Start of encaps. | Middle of encaps. | End of encaps. |
| | Ramipril | | | Indapamide | | |
| 1 | 104.8 | 103.1 | 101.4 | 99.6 | 99.3 | 98.0 |
| 2 | 104.5 | 100.3 | 101.1 | 100.5 | 97.1 | 97.8 |
| 3 | 102.0 | 101.2 | 103.2 | 98.2 | 97.7 | 99.9 |
| Av | 103.8 | 101.5 | 101.9 | 99.4 | 98.0 | 98.6 |
| RSD | 1.5 | 1.4 | 1.1 | 1.2 | 1.2 | 1.2 |

As can be seen the capsules filled with compositions of the invention are uniform in the context of content of active substance throughout the mixing and encapsulation process.

### Dissolution profile

In the following tables, the dissolution profile of ramipril and indapamide in the capsules of the invention is shown. Tests were conducted in following conditions: Baskets apparatus (USP Apparatus 1), QC dissolution medium: 0.1M HCl; medium volume 900mL; the rotation 100rpm. Dissolution test is performed according to procedure described in Ph.Eur. Section 2.9.3.

In the case of ramipril, Delix^{®} Protect 10 mg and Triatec^{®} 10 mg ramipril tablets were also tested. For indapamide, Natrilix^{®} 2.5 mg indapamide tablets were tested for comparison. All the tests were performed for 6 tablets or 6 capsules, respectively

| Time (min) | **% Dissolution ramipril** | | | | | | | |
|---|---|---|---|---|---|---|---|---|
| | **Capsules 1** | | **Capsules 2** | | **Delix^{®} Protect** | | **Triatec^{®}** | |
| | Mean | RSD | Mean | RSD | Mean | RSD | Mean | RSD |
| 5 | 97.9 | 2.0 | 96.9 | 4.1 | 97.8 | 0.5 | 66.7 | 10.9 |
| 10 | 100.1 | 0.6 | 101.3 | 0.8 | 97.8 | 0.3 | 87.2 | 7.0 |
| 15 | 100.4 | 0.7 | 101.6 | 0.7 | 97.8 | 0.3 | 93.6 | 4.4 |
| 30 | 100.5 | 0.9 | 101.9 | 0.7 | 97.8 | 0.3 | 98.6 | 2.4 |
| 45 | 100.5 | 0.8 | 101.9 | 0.7 | 98.0 | 0.4 | 100.0 | 1.6 |

| Time (min) | **% Dissolution indapamide** | | | | | |
|---|---|---|---|---|---|---|
| | **Capsules 1** | | **Capsules 2** | | **Natrilix^{®}** | |
| | Mean | RSD | Mean | RSD | Mean | RSD |
| 5 | 66.1 | 3.8 | 62.8 | 7.2 | 4.9 | 21.5 |
| 10 | 83.5 | 3.1 | 84.9 | 3.4 | 17.7 | 17.2 |
| 15 | 88.8 | 2.8 | 89.6 | 2.5 | 38.9 | 14.3 |
| 30 | 91.9 | 2.2 | 93.1 | 1.8 | 77.4 | 1.3 |
| 45 | 93.1 | 2.0 | 94.3 | 1.6 | 84.9 | 0.8 |

### Stability assays

The stability of the capsules filled with compositions of the invention and the comparative formulation were tested under different storage conditions of temperature, humidity and time. The results are shown in the tables below.

| **Capsules 1** | | | | | | | |
|---|---|---|---|---|---|---|---|
| TESTS | REQUIREMENTS | | Initial | 1m / 40°C / 75% RH | 2m / 40°C / 75% RH | 6m / 40°C / 75% RH | 12m / 25°C / 60% RH |
| | At release | Up to end of shelf life | | | | | |
| Appearance I | Hard gelatin capsules with white body and cap with printed 150 | | Hard gelatin capsules with white body and cap with printed 150 | No change | No change | No change | No change |
| Appearance II | content is white or almost white powder or slightly compacted agglomerates | | content is white | No change | No change | No change | No change |
| Average weight (mg) | 205 ± 5% | | 204.8 | 205.6 | 205.3 | 203.8 | 205.4 |

| Related substances by UPLC composition 1 | | | | | | | |
|---|---|---|---|---|---|---|---|
| Impurities | REQUIREMENTS | | Initial | 1m / 40°C / 75% RH | 2m / 40°C / 75% RH | 6m / 40°C / 75% RH | 12m / 25°C / 60% RH |
| | At release | Up to end of shelf life | | | | | |
| ramipril impurity D | NMT 0.5% | NMT 4.0% | 0.16% | 0.44% | 1.18% | 2.48% | 0.42% |
| Single unspecified impurity of ramipril | NMT 0.2% | | BRT | BRT | BRT | BRT | BRT |
| Single unspecified impurity of indapamide | NMT 0.5% | | BRT | BRT | BRT | BRT | BRT |
| Total impurities | NMT 2.0% | NMT 7.0% | 0.17% | 0.44% | 1.18% | 2.48% | 0.42% |
| Assay of ramipril in 1 capsule | 90.0% - 105.0% | | 97.1% | 97.4% | 94.8% | 101.6% | 99.4% |
| Assay of indapamide in 1 capsule | 95.0% - 105.0% | | 99.5% | 99.2% | 99.6% | 99.4% | 98.7% |
| Loss of drying in capsules filling | NMT 6% | | 2.96% | NT | 3.05% | NT | NT |

| | | | | | | | |
|---|---|---|---|---|---|---|---|
| * m: month, RH: relative humidity, UPLC: Ultra-performance liquid chromatographic (UPLC), NMT: not more than, BRT - below reporting threshold (0.10%), NT - not tested | | | | | | | |

| **Comparative capsules 1** | | | | | | | |
|---|---|---|---|---|---|---|---|
| **Impurities** | **Stability conditions** | | **initial** | **2 weeks, 50°C, 75% RH** | **3 months, 40°C, 75% RH** | **6 months, 25°C, 60% RH** | **6 months, 40°C, 75% RH** |
| | at release | shelf-life | | | | | |
| Imp. D Ram | 0.5% | 4.0% | 0.18% | 2.28% | 2.09% | 0.36% | 4.05% |
| Imp. E Ramipril | 0.5% | 1.5% | BRT | BRT | 0.05 | BRT | 0.10 |
| Imp. unk. Indapamide | 0.5% | 0.5% | BRT | BRT | BRT | BRT | BRT |
| Imp. unk. Ramipril | 0.2% | 0.2% | BRT | BRT | BRT | BRT | BRT |
| Sum of imp. | NMT 2% | NMT 7% | 0.18% | 2.28% | 2.14% | 0.36% | 4.15% |
| Assay Ind [%] | 95 - 105 | 95 - 105 | 97.0 % | NT | 97.8 % | 97.9% | 96.2% |
| Assay Ram [%] | 90-105 | 90-105 | 97.3% | NT | 96.6% | 97.4 % | 88.5 % |

| | | | | | | | |
|---|---|---|---|---|---|---|---|
| * Imp: impurity, unk: unknown, NMT: not more than, BRT: Below Reporting Threshold, NT: not tested | | | | | | | |

| **Comparative capsules 2** | | | | |
|---|---|---|---|---|
| **Impurities** | **Stability conditions** | | **12 months, 25°C, 60% RH** | **12 months, 30°C, 65%RH** |
| | at release | shelf-life | | |
| Imp. D Ram | 0.5% | 4.0% | 1.27% | 2.32% |
| Imp. E Ramipril | 0.5% | 1.5% | 0.08% | 0.12% |
| Sum of imp. | NMT 2% | NMT 7% | 2.19 | 3.52 |
| Assay Ind [%] | 95- 105 | 95 - 105 | 97.4% | NT |
| Assay Ram [%] | 90-105 | 90-105 | 96.3% | 95.1% |

| | | | | |
|---|---|---|---|---|
| * Imp: impurity, unk: unknown, NMT: not more than, BRT: Below Reporting Threshold, NT: not tested | | | | |

| **Comparative capsules 3** | | | | | | | |
|---|---|---|---|---|---|---|---|
| **Impurities** | **Stability conditions** | | **initial** | **2 weeks, 50°C, 75% RH** | **3 months, 40°C, 75% RH** | **6 months, 25°C, 60% RH** | **6 months, 40°C, 75% RH** |
| | at release | shelf-life | | | | | |
| Imp. D Ram | 0.5% | 4.0% | 0.25% | 2.85% | 5.14% | 0.57% | 5.16% |
| Imp. E Ramipril | 0.5% | 1.5% | BRT | BRT | 0.15% | BRT | 0.09% |
| Imp. unk. Indapamide | 0.5% | 0.5% | BRT | BRT | 0.11% | BRT | BRT |
| Imp. unk. Ramipril | 0.2% | 0.2% | BRT | BRT | BRT | BRT% | 0.12% |
| Sum of imp. | NMT 2% | NMT 7% | 0.25% | 2.85% | 5.40% | 0.57% | 5.37% |
| Assay Ind [%] | 95 - 105 | 95 - 105 | 97.4% | NT | 96.3% | 97.3% | 96.4% |
| Assay Ram [%] | 90-105 | 90-105 | 95.9% | NT | 92.2% | 95.8% | 91.0% |

| | | | | | | | |
|---|---|---|---|---|---|---|---|
| * Imp: impurity, unk: unknown, NMT: not more than, BRT: Below Reporting Threshold, NT: not tested | | | | | | | |

As can be seen the level of total impurities of the composition of the invention (capsules 1) after storage during 6 months at 40 °C/75% RH was 2.48%, while it was of 4.15% and 5.37% for the comparative compositions 1 and 3, respectively, according to the prior art. Further, the level of total impurities of the composition of the invention after storage during 12 months at 25 °C/75% RH was 0.42%, much lower than the 2.19% for the comparative composition 2 according to the prior art.

### Citation List

Russian patent RU2618471
European Pharmacopoeia 7.0, 2.9.40. Uniformity of dosage units

## Claims

1. A dry powder blend pharmaceutical composition comprising:
a) a therapeutically effective amount of ramipril or a pharmaceutically acceptable salt thereof, or a hydrate thereof;
b) a therapeutically effective amount of indapamide or a hydrate thereof;
c) microcrystalline cellulose;
d) mannitol; and
e) optionally one or more other pharmaceutically acceptable excipients.

2. The dry powder blend pharmaceutical composition according to claim 1, wherein microcrystalline cellulose is present in an amount from 5 to 80%, and mannitol is present in an amount from 5 to 80%, wherein the % are expressed in weight with respect to the total weight of the composition.

3. The dry powder blend pharmaceutical composition according to claim 1 or 2, wherein ramipril or a pharmaceutically acceptable salt thereof, or a hydrate thereof, is present in an amount from 1 to 10%, by weight with respect to the total weight of the composition.

4. The dry powder blend pharmaceutical composition according to any of the claims 1 to 3, wherein indapamide, or a hydrate thereof, is present in an amount from 0.5 to 3%, by weight with respect to the total weight of the composition.

5. The dry powder blend pharmaceutical composition according to any of the claims 1-4, wherein ramipril is present as ramipril in free acid non-solvated (non-hydrated) form.

6. The dry powder blend pharmaceutical composition according to any of the claims 1-5, wherein indapamide is present as indapamide hemihydrate.

7. The dry powder blend pharmaceutical composition according to any of the claims 1-6, wherein the weight ratio between microcrystalline cellulose and mannitol is from 5:0.5 to 0.5:5.

8. The dry powder blend pharmaceutical composition according to any of the claims 1-7, which comprises one or more other pharmaceutically acceptable excipients selected from the group consisting of lubricants, disintegrants, binders, diluents, glidants, and combinations thereof.

9. The dry powder blend pharmaceutical composition according to any of the claims 1-8, which comprises:
a) from 1 to 10% of ramipril or a pharmaceutically acceptable salt thereof, or a hydrate thereof;
b) from 0.5 to 3% of indapamide, or a hydrate thereof;
c) from 5 to 80% of microcrystalline cellulose;
d) from 5 to 80% of mannitol; and
e) a lubricant in an amount from 0.1 to 10%; or alternatively
a) from 1 to 10% of ramipril or a pharmaceutically acceptable salt thereof, or a hydrate thereof;
b) from 0.5 to 3% of indapamide, or a hydrate thereof;
c) from 5 to 80% of microcrystalline cellulose;
d) from 5 to 80% of mannitol;
e) a lubricant in an amount from 0.1 to 10%; and
f) a disintegrant in an amount from 1 to 15%;
wherein the % are expressed in weight with respect to the total weight of the composition.

10. An oral unit dosage form comprising the dry powder blend pharmaceutical composition as defined in any of the claims 1-9.

11. The oral unit dosage form according to claim 10, which is a capsule, preferably it is a hard capsule, more preferably it is a hard gelatine capsule.

12. The oral unit dosage form according to claim 10 or 11, which comprises ramipril or a pharmaceutically acceptable salt thereof, or a hydrate thereof in an amount from 2 to 20 mg, based on ramipril free-acid non-solvated form; and indapamide or a hydrate thereof in an amount from 0.75 to 5 mg, based on indapamide in a non-solvated form.

13. The oral unit dosage form according to any of the claims 10-12, which exhibits a dissolution profile according to which at least 80% by weight of ramipril or a pharmaceutically acceptable salt thereof is dissolved in a 0.1M HCl medium within 15 minutes, and at least 75% by weight of indapamide or a pharmaceutically acceptable salt thereof is dissolved in a 0.1M HCl medium within 15 minutes.

14. A process for the preparation of the dry powder blend pharmaceutical composition as defined in any of the claims 1 to 9, which comprises dry mixing ramipril or a pharmaceutically acceptable salt thereof, or a hydrate thereof; indapamide or a hydrate thereof; microcrystalline cellulose; mannitol; and optionally one or more other pharmaceutically acceptable excipients.

15. The process according to claim 14, wherein the process comprises the following steps:
i) dry mixing ramipril, or a pharmaceutically acceptable salt thereof, or a hydrate thereof, with part 1 of the microcrystalline cellulose;
ii) dry mixing indapamide, or a hydrate thereof with part 2 of the microcrystalline cellulose;
iii) dry mixing the mixture of step i), the mixture of step ii) and part 3 of the microcrystalline cellulose:
iv) dry mixing the mixture of step iii), mannitol, part 4 of the microcrystalline cellulose, and optionally one or more other pharmaceutically acceptable excipients except the lubricant (if present); and
v) dry mixing the mixture of step iv) and the lubricant (if present);
wherein steps i) and ii) may be carried out in any order, and part 1 of the microcrystalline cellulose represents from 2 to 25% of the total microcrystalline cellulose present in the composition, part 2 of the microcrystalline cellulose represents from 2 to 25% of the total microcrystalline cellulose present in the composition, part 3 of the microcrystalline cellulose represents from 2 to 25% of the total microcrystalline cellulose present in the composition, and part 4 of the microcrystalline cellulose represents from 25 to 94% of the total microcrystalline cellulose present in the composition.
